**Europäisches Patentamt**

**European Patent Office**

**Office européen des brevets**

(19)

(11) Veröffentlichungsnummer : **0 355 572 B1**

(12)

# EUROPÄISCHE PATENTSCHRIFT

(45) Veröffentlichungstag der Patentschrift :
**17.03.93 Patentblatt 93/11**

(51) Int. Cl.⁵ : **C07K 5/06,** C07K 17/12,
// C07K3/18, C12N9/64,
C12N9/72

(21) Anmeldenummer : **89114645.8**

(22) Anmeldetag : **08.08.89**

(54) **Peptidderivate, Verfahren zu ihrer Herstellung und ihre Verwendung.**

(30) Priorität : **12.08.88 DE 3827415**

(43) Veröffentlichungstag der Anmeldung :
**28.02.90 Patentblatt 90/09**

(45) Bekanntmachung des Hinweises auf die
Patenterteilung :
**17.03.93 Patentblatt 93/11**

(84) Benannte Vertragsstaaten :
**AT BE CH DE FR GB IT LI LU NL SE**

(56) Entgegenhaltungen :
**EP-A- 0 167 152
CHEMICAL ABSTRACTS, vol. 108, 1988, page
324, abstract no. 90917e, Columbus, Ohio, US;
S. SOEDA et al.: "Localization of the binding
sites of porcine tissue-type plasminogen activator and plasminogen to heparin", &
BIOCHIM. BIOPHYS. Acta 1987, 916(3), 279-87**

(73) Patentinhaber : **BEHRINGWERKE
Aktiengesellschaft
Postfach 1140
W-3550 Marburg 1 (DE)**

(72) Erfinder : **Stüber, Werner, Dr.
Cölber Weg 12
W-3551 Lahntal (DE)**
Erfinder : **Pâques, Eric Paul, Dr.
Schmiedeacker 18
W-3550 Marburg (DE)**

(74) Vertreter : **Fischer, Hans-Jürgen, Dr. et al
Hoechst AG Zentrale Patentabteilung
Postfach 80 03 20
W-6230 Frankfurt am Main 80 (DE)**

**Beschreibung**

Die Erfindung betrifft Peptidderivate der Formel I

R-Y-Leu-Pro-NH-CH(CH$_2$-CH$_2$-CH$_2$-NH-C(=NH)-NH$_2$)-X

worin Y HN-(CH$_2$)$_n$-CO mit n = 1 bis 8 oder eine Bindung, X CN, CH$_2$OH oder CHO und R ein zur Affinitätschromatografie geeigneter Trägermatrix oder ein Wasserstoffatom sind, ein Verfahren zu ihrer Herstellung und ihre Verwendung zur Affinitätschromatographie besonders zur Isolierung und Reinigung von Proteasen.

In den letzten Jahren wurde die Isolierung von Proteasen durch die Technik der Affinitätschromatographie wesentlich verbessert. Diese Methode nutzt spezifische Wechselwirkungen zwischen Substanzen aus. Dazu wird eine Substanz als Ligand an einen unlöslichen Träger (Matrix) kovalent gebunden. Der Ligand muß mit der zu isolierenden Substanz in eine komplexartige Wechselwirkung treten können. Der Ligand hält nur die Substanzen zurück, die spezifisch mit ihm reagieren. Andere Stoffe werden ausgewaschen. Die zurückgehaltenen Substanzen können vom Trägermaterial mittels einer Lösung von ungebundenem Liganden oder beispielsweise mit einem Salzgradienten eluiert werden.

Zur Isolierung eines Proteins ist es natürlich am günstigsten, einen Liganden zu benutzen, der nur mit einem oder wenigen Proteinen Wechselwirkungen eingeht. Die Kapazität des Adsorbens ist von einer genügend hohen Ligandenbeladung der Matrix abhängig. Die chemische Bindung sollte möglichst einheitlich und stabil, das heißt schwer hydrolysierbar sein. Die Affinitätschromatographie läßt sich besonders zur Gewinnung von Proteasen, beispielweise aus Plasma, Serum, Körperflüssigkeiten, Zellen oder Zellkulturüberständen einsetzen. Als Affinitätsmaterialien haben sich dabei immobilisierte natürlich vorkommende oder synthetisch hergestellte Inhibitoren bewährt. Der Einsatz der bis jetzt beschriebenen Affinitätsmaterialien ist jedoch aufgrund der eingesetzten Inhibitoren durch erhebliche Mängel eingeschränkt.

Niedermolekulare Inhibitoren wie Arginin oder Benzamidin weisen eine für die Affinitätschromatographie nicht ausreichend starke inhibitorische Aktivität auf. Folglich werden große Harzmengen aufgrund der niedrigen Kapazität benötigt. Im Gegensatz dazu zeigen polypeptidische Inhibitoren wie Aprotinin, SBTI, BPTI, Hirudin oder Eglin eine zu starke inhibitorische Wirkung, so daß die am Affinitätsharz gebundene Protease nur unter extrem starken Bedingungen und/oder mit erheblichem Ausbeuteverlust eluiert werden kann.

Der vorliegenden Erfindung lag die Aufgabe zugrunde, ein Material zur Affinitätschromatographie mit großer Kapazität, Ligandendichte und Stabilität herzustellen, das für die Isolierung von Proteinasen eingesetzt werden kann.

In EP-A-0167152 ist das Peptidderivat D-Val-Gly-DL-Arginal gebunden an einen Träger als Affinitätsmaterial beschrieben. Dieses Material ist jedoch nicht optimal und führt, wie in den Beispielen dieser Anmeldung angegeben, zu Ausbeuteverlusten in Höhe von etwa 30 %.

Überraschenderweise wurde nun gefunden, daß dieser Nachteil durch den Ersatz der Val-Gly-Arginal-Sequenz durch die Sequenzen Leu-Pro-Arginal, D-Leu-Pro-Arginal, L-Leu-Pro-DL-Arginal oder DL-Leu-Pro-DL-Arginal behoben werden kann. Ebenfalls kann das Aldehyd-Derivat Arginal gegen ein entsprechendes Nitrilderivat oder Alkoholderivat ausgetauscht werden.

Gegenstand der Erfindung ist somit eine Verbindung der Formel I.

Falls R einen Träger bedeutet, ist dieser vorzugsweise ein Polymer, das auf Kohlenhydratbasis oder auf Basis von Methacrylamid, N-Methylen-bis-methacrylamid, Glycidylmethacrylat-Polyethylenglykolderivaten und Pentaerythritdimethacrylat aufgebaut ist, besonders $^R$Sepharose, $^R$Biogel, $^R$Sephadex, $^R$Cellex oder $^R$Fractogel.

Gegenstand der Erfindung ist auch Verfahren zur Herstellung einer Verbindung der Formel I, dadurch gekennzeichnet, daß geschützte Aminosäuren oder Peptidsegmente nach an sich bekannten Methoden zu den entsprechenden Peptiden aufgebaut werden, wobei der C-Terminus des Arginins in eine Alkohol-, Aldehyd- oder Nitrilfunktion überführt wird und gegebenenfalls dieses Peptid nach Abspaltung der Schutzgruppen an ein Polymer gebunden wird.

Zur Herstellung einer erfindungsgemäßen Verbindung, die am C-terminalen Ende eine Alkoholgruppe bzw. Aldehydgruppe trägt, nach an sich bekanntem Verfahren, wird die Synthese vorzugsweise mit einem Argininester, vorzugsweise einem Methyl- oder Ethylester, dessen Guanidinogruppe mit einer in der Peptidchemie üblicherweise verwendeten Schutzgruppe blockiert sein kann, vorzugsweise jedoch durch Protonierung geschützt ist, begonnen, indem repetitiv und unter Reinigung der Zwischenstufen einzelne, geschützte Aminosäurederivate, vorzugsweise ein geschütztes Peptidsegment, an die N-alpha-Gruppe des Argininderivates gekoppelt wird. Diese Peptidsegmente weisen die Strukturen Sch-Y-Leu-Pro-OH auf, wobei Sch eine Schutzgruppe, vorzugsweise Boc ist und Y die oben angegebene Bedeutung hat. Leucin weist die D-, L- oder DL-, bevorzugt jedoch die D-Form und Prolin die L-Form auf. Die Synthese erfolgt in Lösung, indem ein Prolinester, vorzugsweise der Methyl- oder Benzylester, mit einem am N-Terminus geschützten Leucinderivat, wobei als Schutzgruppe vorzugsweise die Boc-Gruppe dient, unter Bildung eines Dipeptids umgesetzt wird. Als Lösungsmittel eignen sich vorzugsweise Dimethylformamid, Dimethylsulfoxid, Dichlormethan, Chloroform, N-Methylpyrrolidon sowie deren Mischungen. Die Bildung der Peptidbindung wird durch die Aktivierung der Carboxylgruppe erreicht, wozu die üblicherweise in der Peptidchemie benutzten Aktivierungsmethoden geeignet sind, vorzugsweise die Aktivestermethode und besonders bevorzugt die Carbodiimidmethode unter Zusatz von HOBt und gegebenenfalls einer Base, vorzugsweise N-Methylmorpholin. Die Reinigung der Peptide erfolgt durch Extraktion aus wässriger Lösung mit einem Lösemittel, das in Wasser schlecht löslich ist, vorzugsweise mit Ethylacetat.

Gegebenenfalls wird die N-terminale Schutzgruppe entfernt und nach derselben Arbeitsmethode Boc-geschütztes Y-OH, wobei Y-OH vorzugsweise epsilon-Aminocapronsäure ist, an das Dipeptid gekoppelt. Die Estergruppe am Prolin wird durch Verseifung oder Hydrogenolyse entfernt. Die Reaktionsführung bei der Segmentkopplung an das Argininderivat entspricht vorzugsweise der Dipeptidherstellung. Das Dipeptidderivat wird daraufhin in einem Alkohol, vorzugsweise Ethanol, gelöst und in Gegenwart von $CaCl_2$ mit Natriumborhydrid reduziert. Eine bevorzugte Ausgestaltung der Reduktion sieht dabei vor, 20 g Boc-Aca-D-Leu-Pro-Arg-OMe in 200-500 ml Ethanol zu lösen, 7-15 g $CaCl_2.2H_2O$, vorzugsweise 11 g darin zu lösen und den Methylester mit 5 - 10 g, vorzugsweise 6,5 g Natriumborhydrid zu reduzieren. Die Reaktionstemperatur wird im Bereich von 4 bis 50° C während 30 min bis 6 Stunden, vorzugsweise bei Raumtemperatur während 1 Stunde gehalten. Das reduzierte Peptid wird durch Extraktion mit einem mit Wasser nicht mischbaren Lösemittel, vorzugsweise Butanol aus der Wasserphase gewonnen. Gegebenenfalls werden an dieser Stufe vorhandene Schutzgruppen, vorzugsweise acidolytisch, entfernt.

Die Herstellung der erfindungsgemäßen Arginaldivate wird vorzugsweise durch Partialoxidation der entsprechenden Arginolderivate ausgeführt, wie sie in Chem. Pharm. Bull. 17 (9), 1902 - 1909 (1960) beschrieben ist.

Bei einer bevorzugten Ausführung dieser Oxidation werden 5 g Boc-Aca-D-Leu-Pro-Arg-OH in 80-120 ml Dimethylsulfoxid gelöst und mit 6 - 12 g, vorzugsweise 8 g Dicyclohexylcarbodiimid, und 500 mg - 1500 mg, vorzugsweise 850 mg, wasserfreier kristalliner ortho-Phosphorsäure umgesetzt. Die Reaktionszeit beträgt 6 - 24 Stunden, vorzugsweise 14 Stunden, bei Raumtemperatur. Das Rohpeptid wird an [R]Sephadex-LH 20 in Methanol chromatographiert.

Die vorzugsweise am N-Terminus eingesetzte Boc-Schutzgruppe wird dann gegebenenfalls acidolytisch, bevorzugt mit einer Mischung aus 50 % Trifluoressigsäure, 45 % Methylenchlorid und 5 % Anisol, entfernt. Die Herstellung eines Peptids, das eine erfindungsgemäße Peptidsequenz aufweist und dessen C-terminale Gruppe eine Cyanogruppe besitzt, erfolgt vorzugsweise ebenfalls mittels der obengenannten Peptidsegmente der allgemeinen Struktur Sch-Y-Leu-Pro-OH, worin Sch eine Schutzgruppe, vorzugsweise Fmoc, und Y eine Bindung oder $NH-(CH_2)_n-CO$ mit n = 1 bis 8, vorzugsweise n = 6 ist.

Als C-terminaler Baustein wird Arg-CN benutzt, dessen Herstellung in Int. J. Pept. Prot. Res. 31, 63 - 70 (1988) beschrieben ist.

Die N-terminale Schutzgruppe, vorzugsweise Fmoc, wird mit einer Base, bevorzugt 20 % Piperidin in DMF, abgespalten und das Peptidnitril durch Chromatographie an Sephadex [(R)]-LH 20 gereinigt.

Die erfindungsgemäßen Peptide zeichnen sich durch eine starke inhibitorische Wirkung auf t-PA aus. Darüber hinaus eignen sich diese Peptide überraschenderweise zur Affinitätschromatographie des t-PA, wenn diese Peptide an ein Polymer gebunden sind, das mit einer t-PA-haltigen Lösung in Kontakt gebracht wird. Ganz besonders überraschend war es, daß immobilisierte Peptide mit einer Gruppe Y, wie oben beschrieben, mit n bevorzugt = 6, eine deutlich höhere Bindungsaffinität zu t-PA hatten als solche immobilisierten Peptide, bei denen Y eine Bindung ist.

Gegenstand der Erfindung ist auch die Verwendung dieser Peptide zur Reinigung von Proteasen, vorzugsweise Serin-und Cysteinproteasen, besonders bevorzugt Gewebsplasminogen-Aktivator, Prourokinase, Urokinase, deren natürlich vorkommenden, synthetisch oder gentechnisch hergestellten Derivaten aus Plasma, Serum, Körperflüssigkeiten, Zellen oder Zellkulturüberständen.

Bevorzugt wird ein Verfahren zur Reinigung von Proteasen dadurch gekennzeichnet, daß man das Ausgangsmaterial mit einer Leitfähigkeit von 0 bis 100 mSi und einem pH-Wert von 3 bis 11, vorzugsweise mit einer Leitfähigkeit von 0 bis 30 mSi und einem pH-Wert von 5 bis 8 in Kontakt mit dem Affinitätsmaterial bringt. Das Material wird mit einer gepufferten Lösung (pH 3 bis 11; Leitfähigkeit 0 bis 100 mSi, vorzugsweise pH 4 bis 8 und Leitfähigkeit 0 bis 30 mSi) gewaschen und anschließend die Protease mit einer gepufferten Neutralsalzlösung eluiert. Auf diese Weise können Proteasen, die mit der Peptidsequenz der Verbindungen der Formel I in Wechselwirkung treten, aus Lösungen adsorbiert und gegebenenfalls dann eluiert werden, beispielsweise t-PA, Prourokinase und Urokinase. Wurde ein nach der beschriebenen Methode hergestelltes Affinitätsmaterial angewendet, zeigte sich eine höhere Bindungskapazität und eine Wiederfindungsrate von über 90 % anstatt von 75 % für t-PA im Vergleich zu einem Material, das nach EP-A-0 167 152 aus Val-Gly-Arginal und einem unlöslichen Trägermaterial hergestellt wurde.

Abkürzungen

| | |
|---|---|
| SBT I | Soya-bean trypsin inhibitor |
| BPT I | bovine pancreas trypsin inhibitor |
| DMF | Dimethylformamid |
| Fmoc | 9-Fluoroenylmethyloxycarbonyl |
| Boc | Butyloxycarbonyl |
| HOBt | Hydroxybenzotriazol |
| Arg | Arginin |
| Pro | Prolin |
| Leu | Leucin |
| Gly | Glycin |
| Val | Valin |
| t-PA | Gewebsplasminogen-Aktivator |
| mSi | milli-Siemens |
| DCC | Dicyclohexylcarbodiimid |
| DCU | Dicyclohexylharnstoff |
| Z | Benzyloxycarbonyl |

Chirale Aminosäuren können in der D-oder L-Form vorliegen. Bei Fehlen einer Angabe ist die L-Form gemeint.

Beispiele

Beispiel 1

Herstellung von D-Leu-Pro-Arginol

4,3 g Boc-Pro, 2,7 g HOBt und 4,2 g DCC wurden in 50 ml DMF gelöst und nach 1 Stunde wurden 5,22 g Argininmethylesterdihydrochlorid und 2,2 ml N-Methylmorpholin zugesetzt. Der Ansatz wurde über Nacht bei Raumtemperatur gerührt, DCU wurde abfiltriert und das Lösemittel im Vakuum abgedampft. Der Rückstand wurde in Ethylacetat gelöst, mit Wasser mehrfach extrahiert und die Wasserphase gefriergetrocknet. Das Rohprodukt wurde in n-Butanol gelöst und mit Wasser dreimal extrahiert. Nach Trocknung der organischen Phase über Natriumsulfat wurde das Lösemittel abgedampft. 7,1 g Boc-Pro-Arg-OMe wurden 1 Stunde mit 150 ml 1,2 N HCl/Eisessig bei Raumtemperatur behandelt und das Abspaltungsmittel im Vakuum abdestilliert. Nach Trocknung des öligen Rückstandes wurde 4 g Boc-D-Leu mit 2,4 g HOBt und 3,64 g DCC in 50 ml DMF 1 Stunde lang aktiviert und zu dem Dipeptid gegeben. Nach Zusatz von 1,9 ml N-Methylmorpholin wurde der Reaktionsansatz über Nacht bei Raumtemperatur gerührt, Unlösliches durch Filtration entfernt und das Lösemittel abgedampft. Der ölige Rückstand wurde zwischen Ethylacetat und Wasser verteilt, die Ethylacetatphase noch zweimal mit kleinen Wasserportionen extrahiert und die Wasserphase gefriergetrocknet. Das Rohprodukt wurde an $^R$Sephadex-LH 20 in Methanol chromatographiert (Ausbeute 5,8 g). 19,5 g von diesem Methylesterderivat und 2,16 g CaCl$_2 \cdot$ 2H$_2$0 wurden in 100 ml Ethanol bei Raumtemperatur gelöst und 0,8 g Natriumborhydrid in 4 Portionen zu je 200 mg zugesetzt. Nach 1 Stunde wurde das Lösemittel abgedampft, der Rückstand in wenig Wasser aufgenommen und das Produkt mit Butanol extrahiert. Nach Trocknung der Butanollösung mit Natriumsulfat wurde das Lösemittel abgedampft-und der Rückstand an $^R$Sephadex-LH 20 in Methanol chromatographiert (Ausbeute 1,12 g).

1 g Boc-D-Leu-Pro-Arg-CH$_2$OH wurde in 50 ml Trifluoressigsäure/ Dichlormethan/ Anisol (45:50:5) gelöst und 15 min bei Raumtemperatur gerührt. Daraufhin wurde das Lösemittel im Vakuum abgedampft, der Rückstand in Wasser aufgenommen, mit Diethylether zweimal extrahiert und die Wasserphase gefriergetrocknet (Ausbeute 835 mg).

Aminosäureanalyse: Leu 1,00; Pro 1,04; Arg 0,04.

Beispiel 2

Herstellung von epsilon-Aca-D-Leu-Pro-Arg-CHO

9,3 g Boc-epsilon-Aca, 5,4 g HOBt und 8,4 g DCC wurden 1 Stunde lang in 100 ml DMF gerührt. Dann wurden 19 g D-Leu-Pro-Arg-OMe x 2 HCl (Herstellung siehe Beispiel 1: die Boc-Gruppe von Boc-D-Leu-Pro-Arg-OMe wurde mit 1,2 N HCl/Eisessig abgespalten) und 4,4 ml N-Methylmorpholin zugesetzt und über Nacht gerührt. Unlösliches wurde abfiltriert, das Lösemittel abdestilliert und der Rückstand in Wasser aufgenommen. Die Wasserphase wurde zweimal mit Ethylacetat ausgeschüttelt und gefriergetrocknet. Das Rohprodukt wurde in Oberphase gelöst (500 ml Wasser, 400 ml n-Butanol und 100 ml Eisessig intensiv mischen und im Scheidetrichter trennen; die entstandenen Phasen wurden als Ober-/Unterphase benutzt) und mehrfach mit der Unterphase extrahiert. Die Oberphase wurde ankonzentriert und mit Diethylether kristallisiert. 17,3 g dieses Produktes wurden in 400 ml Ethanol gelöst, 10,3 g CaCl$_2$ x 2 H$_2$O und portionsweise 5,9 g Natriumborhydrid zugesetzt. Es wurde 1 Stunde bei Raumtemperatur gerührt und das Lösemittel dann im Vakuum abdestilliert. Der Rückstand wurde in Wasser aufgenommen und mehrfach mit n-Butanol extrahiert. Die organische Phase wurde über Natriumsulfat getrocknet und einrotiert. 9,2 g von Boc-epsilon-Aca-D-Leu-Pro-Arg-CH$_2$OH wurden in 200 ml Dimethylsulfoxid gelöst und mit 13,95 g DCC und 1,5 g wasserfreier Phosphorsäure umgesetzt. Nach 4 Stunden Reaktionszeit bei Raumtemperatur wurde das Lösemittel im Vakuum abgedampft, der Rückstand in Methanol aufgenommen und an [R]Sephadex-LH 20 chromatographiert. 8,1 g des Boc-geschützten Aldehydpeptids wurden in 40 ml Dichlormethan, 4 ml Anisol und 40 ml Trifluoressigsäure gelöst und 15 min bei Raumtemperatur gerührt. Daraufhin wurde der Ansatz durch Eintropfen in Diethylether kristallisiert, die Kristalle gesammelt und im Hochvakuum getrocknet (Ausbeute 8,0 g).

Beispiel 3

Herstellung von epsilon-Aca-D-Leu-Pro-Arg-CN

8,6 g Prolin-tert.-butylester, 13,25 g Z-D-Leucin, 8 g HOBt und 10,5 g DCC wurden in 100 ml DMF gelöst und über Nacht bei Raumtemperatur gerührt. Unlösliches wurde abfiltriert und das Lösemittel im Vakuum abdestilliert. Der ölige Rückstand wurde in Ethylacetat gelöst und je dreimal mit gesättigter Natriumhydrogencarbonatlösung, 5 % Kaliumhydrogensulfatlösung und gesättigter Kochsalzlösung gewaschen. Die organische Phase wurde über Natriumsulfat getrocknet, das Lösemittel entfernt und die Z-Schutzgruppe in Methanol mittels Pd/C und Wasserstoff entfernt. Durch Zusatz von HCl/Methanol wurden die freien Aminogruppen in das Hydrochlorid überführt. Nach Entfernung des Lösemittels wurde Leu-Pro-tert.-butylester aus Methanol/Diethylether kristallisiert. 12,2 g dieses Produktes wurden mit 8,1 g HOBt, 14,1 g Fmoc-Aca, 9,1 g DCC und 4,4 ml N-Methylmorpholin in 150 ml DME über Nacht bei Raumtemperatur gerührt. Nach Abfiltration von DCU wurde das Produkt durch Zusatz von Wasser ausgefällt. Die Kristalle wurden gesammelt und im Hochvakuum über P$_4$O$_{10}$ getrocknet. 19,4 g des t-Butylesters wurden mit 150 ml 1,2 N HCl/Eisessig 1 Stunde bei Raumtemperatur gerührt und das Abspaltungsmittel im Vakuum abgedampft. Der Rückstand wurde aus Methanol/Diethylether umkristallisiert. Dieses Produkt wurde mit 6,7 g HOBt und 6,8 g DCC in 150 ml DMF 1 Stunde bei Raumtemperatur gerührt. Daraufhin wurden nach Entfernung des DCU 12,25 g Arg-CN x 2 TFA (Int. J. Peptide Prot. Res. 31, 63 (1988)) und 3,8 ml N-Methylmorpholin zugesetzt. Nach Rühren über Nacht wurde das Lösemittel abgedampft und das Rohprodukt portionsweise an [R]Sephadex-LH 20 in Methanol chromatographiert. 13,2 g Fmoc-epsilon-Aca-D-Leu-Pro-Arg-CN wurden 30 min mit 20 % Piperidin/DMF (V/V) bei Raumtemperatur gerührt und daraufhin wurde das Abspaltungsreagenz im Vakuum abgedampft. Das Rohprodukt wurde in Methanol gelöst, mit HCl/Methanol angesäuert und durch Eintropfen in Diethylether kristallisiert (9,3 g). Aminosäureanalyse: Aca 0,93; Leu 0,95; Pro 1,05; Arg 1,04

Beispiel 4

Immobilisierung der Peptide an Bromcyan-aktivierte [R]Sepharose

10 g Bromcyan-aktivierte [R]Sepharose (Pharmacia) wurden in 100 ml 0,001 N HCl suspendiert, auf einer Filterfritte mit 500 ml 0,001 N HCl gewaschen und danach portionsweise mit 500 ml 0,25 M Natriumhydrogencarbonatlösung gewaschen. 1,5 g Peptid aus Beispiel 2 oder 3 wurden in 30 ml 0,25 M Natriumhydrogencarbonatlösung gelöst und zum Harz gegeben. Der Ansatz wurde über Nacht bei 4°C geschüttelt, abgesaugt und mit 500 ml 0,1 M Natriumhydrogencarbonat gewaschen. Das Harz wurde mit 100 ml 0,1 Ethanolamin pH 8 2 Stunden bei Raumtemperatur inkubiert und daraufhin 3 x abwechselnd mit 600 ml 0,1 M Natriumacetatlösung pH 4 und 600 ml 0,1 M Natriumhydrogencarbonat pH 8,4 gewaschen. Das Harz wurde zum Schluß mit Wasser ausgewaschen.

Beispiel 5

1. Ausgangslösungen: (AM)

a) Überstand einer t-PA-produzierenden Bowes Melanoma Zellkultur (Eur. J. Biochem. 132, 681-686, 1983)

b) Überstand einer CHO-Zellkultur, die ein deglykosyliertes t-PA-Molekül nach EP-A-0 227 462 (Mutante I) produziert

c) Überstand eines t-PA-wild-typs nach GB 2119804

d) Humaner Urin nach Dialyse gegen 50 mM Tris, 100 mM NaCl pH 7.0

2. Affinitätsharze: (H)

1. Sepharose-Aca-D-Leu-Pro-Arginal (Beispiele 2/4)

2. Sepharose-Aca-D-Leu-Pro-Argininnitril (Beispiele 3/4)

3. Sepharose-L-Val-Gly-DL-Arginal nach EP-A-0 167 152

3. Durchführung:

10 ml der jeweiligen Ausgangslösungen (a-d) wurden jeweils mit 2 g Affinitätsharz (1-3) versetzt, 30 min. bei RT inkubiert. Anschließend wurde das Harz abgetrennt und die Antigenkonzentration mittels ELISA im Überstand bestimmt.

4. Ergebnisse:

|     |     | AM  |     |     |
| --- | --- | --- | --- | --- |
| H   | a   | b   | c   | d   |
| 1   | 5 % | 6 % | 4 % | 9 % |
| 2   | 12 % | 11 % | 15 % | 20 % |
| 3   | 35 % | 36 % | 28 % | 45 % |

Beispiel 6

10 l der Lösung b (Beispiel 1) wurde mit 300 g Harz 1 (Beispiel 1) 30 min. bei RT unter Rühren inkubiert. Die Mischung wurde in eine Säule gefüllt und mit 5 l einer Lösung, enthaltend 0.1 M NaCl, 50 mM Glycin pH 7 gewaschen. Anschließend wurde t-PA mit einer Lösung, enthaltend 0.5 M Arginin, 10 mM Essigsäure pH 4.0 eluiert. Die Ausbeute betrug 98 %.

**Patentansprüche**

1. Peptidderivate der Formel I

$$R\text{-}Y\text{-}Leu\text{-}Pro\text{-}NH\text{-}CH(CH_2\text{-}CH_2\text{-}CH_2\text{-}NH\text{-}C(=NH)\text{-}NH_2)\text{-}X \qquad I$$

worin Y HN-$(CH_2)_n$-CO mit n = 1 bis 8 oder eine Bindung,

X CN, $CH_2OH$ oder CHO und

R eine zur Affinitätschromatografie geeignete Trägermatrix oder ein Wasserstoffatom sind.

2. Peptidderivate nach Anspruch 1, dadurch gekennzeichnet, daß die optisch aktiven Zentren alle oder zum Teil in der L-Form vorliegen.

3. Peptidderivate nach Anspruch 1, dadurch gekennzeichnet, daß die Trägermatrix ein Polymer ist.

4. Peptidderivate nach Anspruch 1, dadurch gekennzeichnet, daß der Träger ein Polymer, das auf Kohlenhydratbasis oder auf Basis von Methacrylamid, N-Methylen-bis-methacrylamid, Glycidylmethacrylat-Polyethylenglykolderivaten und Pentaerythritdimethacrylat aufgebaut ist, ist.

5. Peptidderivate nach Anspruch 1, dadurch gekennzeichnet, daß der Träger [R]Sepharose, [R]Biogel, [R]Sephadex, [R]Cellex oder [R]Fractogel ist.

6. Verfahren zur Herstellung einer Verbindung der Formel I, dadurch gekennzeichnet, daß geschützte Aminosäuren oder Peptidsegmente nach an sich bekannten Methoden zu den entsprechenden Peptiden aufgebaut werden, wobei der C-Terminus des Arginins in eine Alkohol-, Aldehyd- oder Nitrilfunktion überführt wird und gegebenenfalls dieses Peptid nach Abspaltung der Schutzgruppen an ein Polymer gebunden wird.

7. Verwendung eines Peptids nach Anspruch 1, wobei R eine zur Affinitätschromatografie geeignete Trägermatrix ist, zur Reinigung einer Protease.

8. Verwendung nach Anspruch 7, dadurch gekennzeichnet, daß die Protease eine Serin- oder Cysteinprotease ist.

9. Verwendung nach Anspruch 7, dadurch gekennzeichnet, daß die Protease ein Gewebsplasminogen-Aktivator, Prourokinase oder Urokinase oder ein natürlich vorkommendes, synthetisch oder gentechnisch hergestelltes Derivat oder aus Plasma, Serum, Körperflüssigkeiten, Zellen oder Zellkulturüberständen gewonnenes Derivat von diesen ist.

## Claims

1. A peptide derivative of the Formula I

   R-Y-Leu-Pro-NH-CH(CH$_2$-CH$_2$-CH$_2$-NH-C(=NH)-NH$_2$)-X          I

   in which Y is HN-(CH$_2$)$_n$-CO with n = 1 to 8 or a bond,
   X is CN, CH$_2$OH or CHO and
   R is a support matrix suitable for affinity chromatography or is a hydrogen atom.

2. A peptide derivative as claimed in claim 1, wherein some or all of the optically active centers are in the L form.

3. A peptide derivative as claimed in claim 1, wherein the support matrix is a polymer.

4. A peptide derivative as claimed in claim 1, wherein the support is a polymer which has a carbohydrate-based structure or a structure based on methacrylamide, N-methylene-bis-methacrylamide, glycidyl methacrylate-polyethylene glycol derivatives and pentaerythritol dimethacrylate.

5. A peptide derivative as claimed in claim 1, wherein the support is [R]Sepharose, [R]Biogel, [R]Sephadex, [R]Cellex or [R]Fractogel.

6. A process for the preparation of a compound of the Formula I, which comprises protected amino acids or peptide segments being built up by methods known per se to give the corresponding peptides, with the C-terminus of arginine being converted into an alcohol, aldehyde or nitrile functionality and, where appropriate, this peptide being bonded to a polymer after elimination of the protective groups.

7. The use of a peptide as claimed in claim 1, where R is a support matrix suitable for affinity chromatography, for purifying a protease.

8. The use as claimed in claim 7, wherein the protease is a serine or cysteine protease.

9. The use as claimed in claim 7, wherein the protease is a tissue plasminogen activator, prourokinase or urokinase or a derivative which is naturally occurring or prepared by synthesis or genetic manipulation, or a derivative thereof obtained from plasma, serum, body fluids, cells or cell culture supernatants.

## Revendications

1. Dérivés peptidiques de formule I

$$R-Y-Leu-Pro-NH-CH(CH_2-CH_2-CH_2-NH-C(=NH)-NH_2)-X \qquad I$$

où

Y est $HN-(CH_2)_n-CO$ avec n = 1 à 8, ou une liaison,

X est CN, $CH_2OH$ ou CHO, et

R est une matrice support adaptée à la chromatographie d'affinité ou un atome d'hydrogène.

2. Dérivés peptidiques selon la revendication 1, caractérisés en ce que les centres optiquement actifs sont présents, totalement ou partiellement, sous la forme L.

3. Dérivés peptidiques selon la revendication 1, caractérisés en ce que la matrice support est un polymère.

4. Dérivés peptidiques selon la revendication 1, caractérisés en ce que le support est un polymère formé à base de glucides ou à base de méthacrylamide, de N-méthylène-bis-méthacrylamide, de dérivés méthacrylate de glycidyle-polyéthylèneglycol et de diméthacrylate de pentaérythritol.

5. Dérivés peptidiques selon la revendication 1, caractérisés en ce que le support est de la $^R$Sepharose, du $^R$Biogel, du $^R$Sephadex, du $^R$Cellex ou du $^R$Fractogel.

6. Procédé pour préparer un composé de formule I, caractérisé en ce qu'on assemble des segments peptidiques ou des aminoacides protégés jusqu'à obtenir les peptides correspondants, selon des méthodes connues en elles-mêmes, en introduisant la terminaison C de l'arginine dans une fonction alcool, aldéhyde ou nitrile, et, éventuellement, en liant ce peptide à un polymère, après avoir éliminé les groupes protecteurs.

7. Utilisation d'un peptide selon la revendication 1, R étant une matrice support adaptée à la chromatographie d'affinité, pour purifier une protéase.

8. Utilisation selon la revendication 7, caractérisée en ce que la protéase est une protéase de la sérine ou une protéase de la cystéine.

9. Utilisation selon la revendication 7, caractérisée en ce que la protéase est un activateur tissulaire du plasminogène, une prourokinase ou une urokinase ou un dérivé de ceux-ci, préparé par voie de synthèse ou par génie génétique, existant à l'état naturel, ou un dérivé de ceux-ci, obtenu à partir du plasma, du sérum, de liquides corporels, de cellules ou de surnageants cellulaires.